# EUROPÄISCHE PATENTSCHRIFT

(11) **EP 2 275 526 B1**
(45) Veröffentlichungstag und Bekanntmachung des Hinweises auf die Patenterteilung: **17.04.2013**
(21) Anmeldenummer: 09011906.6
(22) Anmeldetag: 18.09.2009
(51) Int. Cl.: C12M 1/04, C12M 1/107

(54) **Vorrichtung und Verfahren zur Gewinnung von Biogas**
Device for generating biogas
Dispositif de production de biogaz

(30) Priorität: 13.07.2009 EP 09009128
(43) Veröffentlichungstag der Anmeldung: 19.01.2011
(73) Patentinhaber: KOMPOFERM GmbH, 33428 Marienfeld (DE)
(72) Erfinder: Eggersman, Karlgünter, 33428 Marienfeld (DE)
(74) Vertreter: Schober, Mirko

(56) Entgegenhaltungen:
- EP-A1- 1 428 868
- EP-A1- 1 736 535
- WO-A2-02/06439
- DE-A1- 19 719 323
- DE-A1-102006 009 165
- DE-B3-102005 037 452
- DE-U1- 29 902 143
- DE-U1-202006 002 757
- US-A- 5 269 634

## Beschreibung

Die Erfindung betrifft eine Vorrichtung und ein Verfahren zur Gewinnung von Biogas nach den Oberbegriffen der Ansprüche 1 bzw. 9.

Eine entsprechende Vorrichtung bzw. ein entsprechendes Verfahren ist aus DE 10 2006 009 165 A1 bekannt. Bei der Methanisierung von Biomasse werden Fermenter eingesetzt, in denen ein Gärprozess abläuft, wobei durch das Vergären der Biomasse methanhaltiges Gas entsteht. Man unterscheidet dabei das sogenannte kontinuierliche Verfahren und das diskontinuierliche Verfahren (Batch-Verfahren). Bei letzterem wird ein Mikroorganismen enthaltendes Perkolat in einen Fermenter eingebracht, dieses durchläuft das Substrat (Biomasse) und wird in einem Perkolatbehälter gesammelt und ggf. erneut in den Fermenter eingebracht, bis die im Fermenter enthaltene Biomasse vergoren ist. Die vergorene Biomasse wird anschließend aus dem Fermenter entnommen und muss ggf. aufbereitet und üblicherweise verwertet werden. Um die Verwertung zu ermöglichen, kann es erforderlich sein, dass die vergorene Biomasse hygienisiert ist. Das bedeutet, dass die Biomasse frei von unerwünschten Keimen, Bakterien oder sonstigen Verunreinigungen sein muss, damit der Gärrest verwertet werden kann.

Gleiches gilt auch für das Perkolat, welches aufgrund des Durchspülens der Biomasse ebenfalls entsprechende Bakterien und dergleichen enthält und ebenfalls hygienisiert sein muss, um beim Rezyklieren in den Fermenter bereits hygienisierte Biomasse nicht zu reinfizieren.

Aufgabe der vorliegenden Erfindung ist es daher, eine Vorrichtung und ein Verfahren der eingangs genannten Art zur Verfügung zu stellen, mit der beim diskontinuierlichen Betrieb einer Fermentationsanlage ohne Reinfizieren der Biomasse im Fermenter möglich ist.

Gelöst wird diese Aufgabe durch eine Vorrichtung mit den Merkmalen des Anspruchs 1 sowie ein Verfahren mit den Merkmalen des Anspruchs 9. Vorteilhafte Ausführungsformen finden sich in den abhängigen Ansprüchen.

Erfindungsgemäß wird die Verweilzeit des Perkolates im Perkolatspeicher so eingerichtet, dass das Perkolat am Perkolatauslass des Perkolatspeichers hygienisiert ist. Zur Hygienisierung wird dabei bevorzugt das Perkolat auf einer Heizstrecke hinter dem Perkolateinlass des Perkolatspeichers in einen thermophilen Temperaturbereich, bevorzugt größer 50°C aufgeheizt (andere Temperaturen auch unter 50°C sind je nach Gegebenheiten und eingesetzten Mikroorganismen ebenfalls möglich) und durch den Perkolatspeicher geleitet. Hierzu ist vorgesehen, den Strömungsweg zwischen Perkolateinlass und Perkolatauslass mäandrierend, spiral- oder schneckenförmig auszubilden, so dass aufgrund der verlängerten Wegstrecke eine besonders lange Verweilzeit bei einer thermophil eingestellten Temperatur ermöglicht wird, so dass sichergestellt ist, dass am Perkolatauslass auschließlich hygienisiertes Perkolat entnommen werden kann, welches bei Perkolation durch den Fermenter die dortige Biomasse nicht mehr reinfizieren kann. Nach einer bevorzugten Ausführungsform werden zwei Sandfänge eingesetzt, von denen einer ausschließlich mit hygiensiertem Perkolat und einer mit nicht hygiensiertem Perkolat beschickt wird. Die Beschickung erfolgt aus den verwendeten Fermentern abhängig davon, ob der Fermenter an dessen Auslass hygienisiertes oder nicht hygienisiertes Perkolat zur Verfügung stellt. Das im Sandfang für nicht hygienisiertes Perkolat befindliche Perkolat wird dann in den Perkolatspeicher geleitet und dort hygienisiert. Das im Sandfang für hygienisiertes Perkolat befindliche Perkolat kann ebenfalls in den Perkolatspeicher überlaufen und gelangt ebenfalls hygienisiert zum Perkolatauslass. Besonders Vorteilhaft ist es dabei, wenigstens einen Fermenter thermophil zu betreiben, da dann die Hygienisierung realisiert wird.

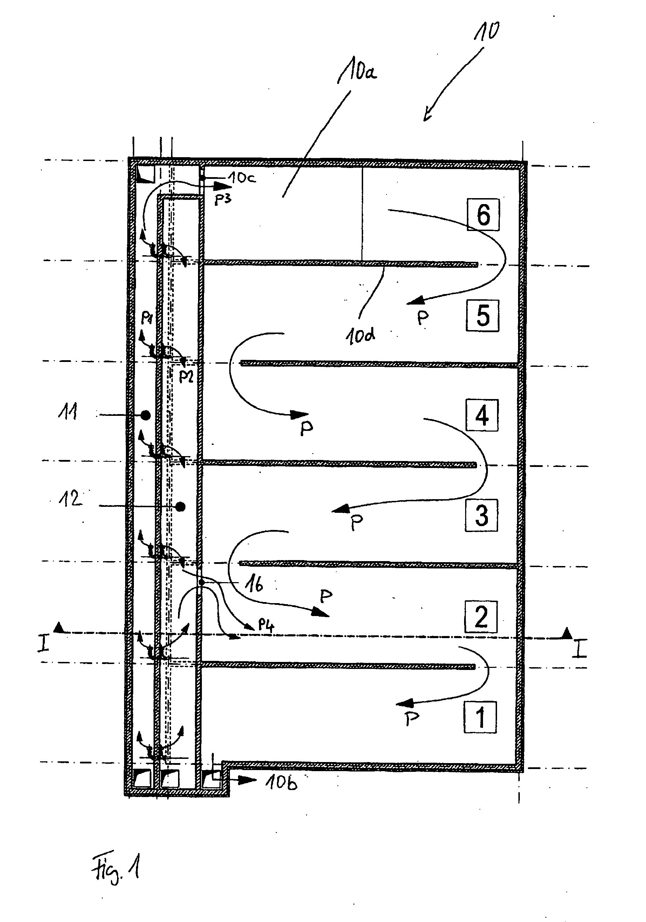

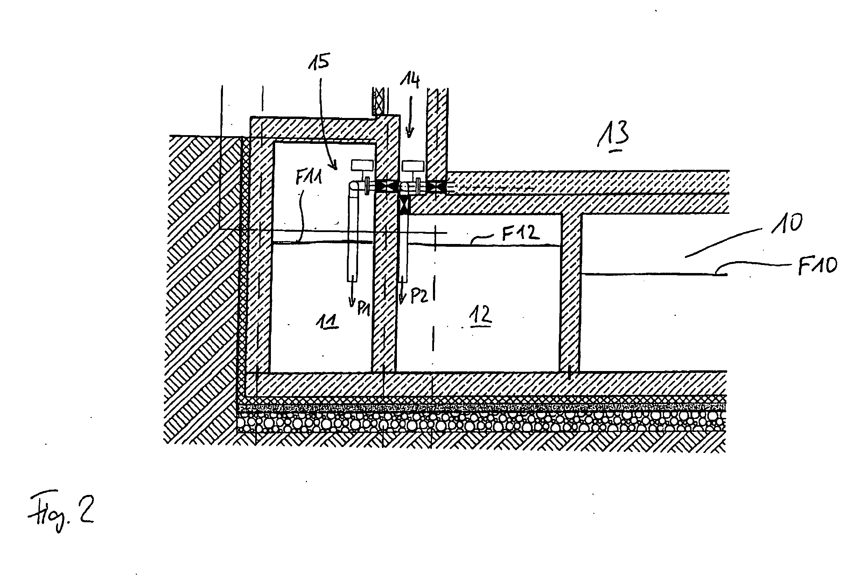

Die Erfindung wird nachfolgend anhand der Zeichnungen der Figuren 1 und 2 schematisch näher erläutert.
- Figur 1: - zeigt einen Teil einer bevorzugten Ausfüh- rungsform für die erfindungsgemäße Vorrichtung in Draufsicht,
- Figur 2: - zeigt eine Schnittansicht durch die in Figur 1 gezeigte Vorrichtung Entlang der Linie I-I in Figur 1.

Die nachfolgende Beschreibung des bevorzugten Ausführungsbeispiels bezieht sich auf beide Figuren gleichermaßen.

Die dargestellte Vorrichtung ist bevorzugt zweigeschossig ausgebildet. Im Obergeschoss (in Figur 1 nicht gezeigt) befinden sich ein oder eine Mehrzahl Fermenter 13 (vgl. Figur 2), in die ein Substrat aus zu vergärender Biomasse eingebracht wird. In der unter den Fermentern 13 liegenden Ebene, die in Figur 1 im Grundriss dargestellt ist, befindet sich ein Perkolatspeicher 10, der bevorzugt eine Mehrzahl Kammern 1-6 aufweist, die im gezeigten Ausführungsbeispiel mittels Wänden 10d voneinander getrennt sind, wobei die Stauwände 10 nicht durchgängig ausgebildet sind, sondern den Durchtritt von Perkolat von einer Kammer (z.B. 6) in die jeweils angrenzende Kammer (z.B. 5) ermöglichen. Bevorzugt sind die Wände 10d so angeordnet, dass sich vom Perkolateinlass 10c in der Kammer 6 zum Perkolatauslass 10b in der Kammer 1 ein durch die Pfeile P angedeuteter, mäandrierender Strömungsweg für das Perkolat ergibt. Andere Formen der Strömungswege, z.B. Spiral oder Schneckenform, sind ebenfalls denkbar.

Des Weiteren befinden sich in dieser Ebene zwei im gezeigten Beispiel als Sandfänge ausgebildete Einrichtungen zur Reinigung des Perkolats 11 und 12. Gezeigt ist ein Sandfang für hygienisiertes Perkolat 12 (im Folgenden "Sandfang 'weiß'" und ein Sandfang für nicht hygienisiertes Perkolat 11 (im Folgenden "Sandfang ,schwarz"'. Wie insbesondere in Figur 2 gezeigt, kann aus den Fermentern 13 entnommenes Perkolat über ein Ventilsystem 14, 15 in den Sandfang 'schwarz' 12 (Weg P1) oder den Sandfang ,weiß' 11 (Weg P2) eingeleitet werden. Aus dem Sandfang 'weiß' 12 kann das Perkolat durch eine Öffnung oder Stauwand 16, die so hoch ausgebildet ist, dass hygienisiertes Perkolat aus dem Sandfang ,weiß' in Richtung des Pfeils P4 in eine Kammer 2 des Perkolatspeichers 10 gelangen kann, jedoch ein Rückfluss aus der Kammer 2 in den Sandfang ,weiß' 12 nicht möglich ist. Dazu wird der Füllstand F10 des Perkolats im Perkolatspeicher 10 unterhalb der Öffnung oder Staumauer 16 bzw. unterhalb des Füllstandes F12 im Sandfang ,weiß' gehalten.

Aus dem Sandfang ,schwarz' 11 gelangt das Perkolat über einen Perkolateinlass 10c, der als Öffnung oder Stauwand ausgebildet sein kann, in den Perkolatspeicher 10 und tritt dort in die Kammer 6 ein. Wie oben liegt auch der Füllstand F11 im Sandfang ,schwarz' bzw. der Perkolateinlass 10c oberhalb des Füllstandes F10 im Perkolatspeicher 10.

Der Betrieb der Anlage läuft nun so ab, dass jeweils Perkolat, welches durch den Einlass 10c in den Perkolatspeicher 10 gelangt (Weg P3), zunächst (hier im ersten, dem Einlass 10c zugewandten Abschnitt 10a) auf einer Heizstrecke erwärmt wird. Bevorzugt wird dabei auf eine Temperatur im thermophilen Bereich erwärmt und sodann mäandrierend (Pfeile P) durch die einzelnen Kammern 6 bis 1 geführt. Die Weglänge P und die Temperatur des Perkolats sind so eingestellt, dass am Perkolatauslass 10b in Kammer 1 ausschließlich hygienisiertes Perkolat entnommen werden kann, welches dann ggf. in die Fermenter 13 zurückgeführt wird. Durch das Zurückführen von "warmen" Perkolat mit einer Temperatur im Bereich von vorzugsweise über 50°C in den Fermenter 13, wird das dort vorhandene Substrat (Biomasse) nach und nach ebenfalls temperiert bis es nach einer bestimmten Zeit (einigen Tagen) ebenfalls wie das Perkolat im Perkolatspeicher eine Temperatur im thermophilen Bereich aufweist. Durch den dann thermophilen Betrieb des betreffenden Fermenters wird auch die darin enthaltene Biomasse hygienisiert, so dass aus dem betreffenden Fermenter 13 am Ende nur noch hygienisiertes Perkolat entnommen und über den Weg P2 in den Sandfang ,weiß' 12 eingeleitet werden kann. Solange aus einem Fermenter 13 noch nicht hygienisiertes Perkolat entnommen wird, wird dieses in den Sandfang ,schwarz' über den Weg P1 geleitet. Von dort aus gelangt dieses Perkolat in den Perkolatspeicher und wird wie oben beschrieben zum Perkolatauslass 10b geführt und so hygienisiert. Bereits hygienisiertes Perkolat aus dem Sandfang ,weiß' 12 wird dem Perkolatkreislauf durch Überlaufen über die Öffnung oder Stauwand 16 zur Verfügung gestellt.

Beispielhaft kann eine entsprechende Vorrichtung etwa wie folgt betrieben werden: Im Fermenter 13 wird Biomasse als Substrat platziert. Typischer Weise verweilt diese im Fermenter 13 zwischen Eintrag der Biomasse bis zu ihrem Austrag nach der Vergärung etwa 21 Tage. Die Materialtemperatur entspricht beim Eintrag den Umgebungsbedingungen, z.B. 10°C. Nun beginnt die Vergärung mit Start der Perkolation mit einer Perkolattemperatur von bevorzugt über 50°C, bevorzugt 53°C, wobei die Perkolattemperatur so geregelt wird, dass sie an der Entnahmestelle 10b des Perkolatspeichers 10 zu jeder Zeit die den genannten Temperaturwert erreicht. Hierzu und zur Einregelung eines entsprechenden Temperaturwertes können im Perkolatspeicher 10 oder/und in einem oder beiden der Sandfänge 11, 12 zusätzliche Heizungen, im Perkolatspeicher 10 insbesondere in den Kammern 1 und 2, vorgesehen sein.

Während dieser Zeit wird der Perkolatablauf aus den Fermentern 13 immer in den Sandfang ,schwarz' 11 (Pfeil P1) geführt. Im Laufe der nächsten Tage (z.B. etwa bis zum 8. oder 9. Tag) wird durch das Perkolieren der Fermenter bzw. der darin enthaltenen Biomasse mit warmen Perkolat eine thermophile Temperatur (bevorzugt > 50°C) des gesamten Materials in dem Fermenter 13 an jeder Stelle erreicht. Ab etwa dem 10. Tag ist damit auch das Material im Fermenter 13 hygienisiert, so dass auch der Perkolatablauf einen 100%igen Hygienestatus aufweist. Ab diesem Zeitpunkt wird das Ventil 14, 15 des Perkolatablaufes so geschaltet, dass der Ablauf in den Sandfang 'weiß' 12 (Pfeil P2) gelangt.

Der Überlauf aus dem Sandfang ,schwarz' 11 gelangt über den Einlass 10c in den Perkolatspeicher 10 im Bereich der Kammer 6. Die Temperatur an dieser Stelle wird durch den Zulauf aus den noch nicht vollständig hygienisierten Inhalten des Fermenters 13 immer weniger als die für die Perkolatentnahme vorgesehene Temperatur (ca. 53°C) betragen. Durch Beheizung der Kammern (insbesondere der Kammer 6 auf der Heizstrecke 10a) wird das Perkolat bevorzugt bis auf 53°C aufgeheizt und auf dem gesamten Strömungsweg bzw. für die gesamte Aufenthaltsdauer auf dieser Temperatur gehalten im Perkolattank 10. Da das Perkolat langsam durch die Kammern 6 bis 1 strömt, ist bevorzugt auf Höhe der Kammern 3 und 2 das Perkolat nach einer definierten Zeit vollständig hygienisiert (z.B. etwa nach 5 bis 8 Tagen Verweildauer gerechnet ab Eintritt in Kammer 6). An dieser Stelle wird nun auch das Perkolat aus dem Überlauf 16 des Sandfang ,weiß' ebenso dem Perkolatspeicher 10 zugeführt, wodurch sich der Volumenstrom nach der Eintrittsstelle erhöht.

Bevorzugt sind die beiden Sandfänge 11, 12 ständig zu 100% bis auf Höhe der Überlaufkante der Öffnungen 16 und 10c gefüllt. Der Wasserstand F10 im Perkolatspeicher 10 muss im Vergleich dazu immer niedriger liegen, um einen Rückfluss vom Perkolatspeicher 10 zurück in die Sandfänge 11, 12 (insbesondere in den Sandfang ,weiß' 12 zu verhindern. Hierzu ist bevorzugt eine Füllstandsüberwachung vorgesehen.

Die hier genannten Temperaturen und insbesondere die genannten Zeiten sind beispielhaft gewählt und können in der Praxis abweichen.

## Patentansprüche

1. Vorrichtung zur Gewinnung von Biogas mit wenigstens einem Fermenter (13) und einem Perkolatspeicher (10) zum Auffangen von aus dem wenigstens einen Fermenter (13) entnommenen Perkolat, wobei der Perkolatspeicher (10) einen mit einem Auslass des wenigstens einen Fermenters (13) verbindbaren Perkolateinlass (10c) und einen Perkolatauslass (10b) aufweist,
**dadurch gekennzeichnet,**
**dass** der Strömungsweg (P) im Perkolatspeicher zwischen dem Perkolateinlass (10c) und dem Perkolatauslass (10b) mäandrierend, spiral- oder schneckenförmig ausgebildet ist, so dass aufgrund des verlängerten Strömungswegs (P) für das Perkolat eine besonders lange Verweilzeit bei thermophil eingestellter Temperatur ermöglicht wird, wobei die Weglänge des Strömungswegs des Perkolats und die Temperatur des Perkolats auf seinem Weg zwischen dem Perkolateinlass (10c) und dem Perkolatauslass (10b) so eingestellt sind, dass am Perkolatauslass (10b) ausschließlich hygenisiertes Perkolat entnommen werden kann.

2. Vorrichtung nach Anspruch 1,
**dadurch gekennzeichnet,**
**dass** der Perkolatspeicher (10) unterhalb des wenigstens einen Fermenters (13) angeordnet ist.

3. Vorrichtung nach einem der vorherigen Ansprüche,
**dadurch gekennzeichnet,**
**dass** eine Mehrzahl Fermenter (13) vorgesehen sind, von denen wenigstens einer für einen wenigstens zweitweisen thermophilen Betrieb ausgelegt ist.

4. Vorrichtung nach einem der vorherigen Ansprüche,
**dadurch gekennzeichnet,**
**dass** zwischen dem wenigstens einen Fermenter (13) und dem Perkolatspeicher (10) eine Einrichtung (11) zur Reinigung des Perkolats geschaltet ist und ggf. eine zusätzliche Einrichtung (12).

5. Vorrichtung nach Anspruch 3 und 4,
**dadurch gekennzeichnet,**
**dass** wenigstens zwei Einrichtungen (11) und (12) zur Reinigung des Perkolats vorgesehen sind, wobei Fermenter (13) und die Einrichtungen (11) und (12) so schaltbar sind, dass aus einem Fermenter (13) kommendes hygienisiertes Perkolat in die eine Einrichtung (12) geleitet und aus einem Fermenter (13) kommendes nicht hygienisiertes Perkolat in die jeweils andere Einrichtung (11) geleitet wird.

6. Vorrichtung nach einem der vorherigen Ansprüche,
**dadurch gekennzeichnet,**
**dass** im Perkolatspeicher (10) hinter dem Perkolateinlass (10c) wenigstens eine Heizstrecke (10a) vorgesehen ist, auf der das in den Perkolatspeicher (10) gelangende Perkolat auf eine vorgegebene Temperatur erwärmbar ist.

7. Vorrichtung nach einem der Ansprüche 4 bis 6,
**dadurch gekennzeichnet,**
**dass** wenigstens eine der Einrichtungen (11) und (12) ein Sandfang ist.

8. Vorrichtung nach einem der vorherigen Ansprüche,
**dadurch gekennzeichnet,**
**dass** im Perkolatspeicher (10) und/oder einer und/oder beiden der Einrichtungen (11, 12) zur Reinigung des Perkolats eine Heizung vorgesehen ist, die so betreibbar ist, dass eine vorgegebene Mindesttemperatur des Perkolats eingeregelt wird.

9. Verfahren zur Gewinnung von Biogas, bei welchem Substrat in wenigstens einem Fermenter (13) mit Perkolat perkoliert wird und das Perkolat nach dem Perkolieren in einen Perkolatspeicher (10) gelangt, wobei der Perkolatspeicher (10) einen mit einem Auslass des wenigstens einen Fermenters (13) verbindbaren Perkolateinlass (10c) und einen Perkolatauslass (10b) aufweist,
**dadurch gekennzeichnet,**
**dass** das Perkolat zwischen dem Perkolateinlass (10c) und dem Perkolatauslass (10b) des Perkolatspeichers (10) auf einem mäandrierenden, schnecken- oder spiralförmigen Strömungsweg (P) geführt wird, so dass aufgrund des verlängerten Strömungswegs (P) für das Perkolat eine besonders lange Verweilzeit bei thermophil eingestellter Temperatur ermöglicht wird, wobei die Weglänge des Strömungswegs (P) des Perkolats und die Temperatur des Perkolats auf seinem Weg zwischen dem Perkolateinlass (10c) und dem Perkolatauslass (10b) so eingestellt sind, dass am Perkolatauslass (10b) ausschließlich hygenisiertes Perkolat entnommen werden kann.

10. Verfahren nach Anspruch 9,
**dadurch gekennzeichnet,**
**dass** eine Mehrzahl Fermenter (13) eingesetzt werden, von denen wenigstens einer wenigstens zeitweise thermophil betrieben wird.

11. Verfahren nach einem der Ansprüche 9 und 10,
**dadurch gekennzeichnet,**
**dass** zwischen dem wenigstens einen Fermenter (13) und dem Perkolatspeicher (10) das Perkolat durch eine Einrichtung (11) zur Reinigung des Perkolats und ggf. eine zusätzliche Einrichtung (12) geführt wird.

12. Verfahren nach Anspruch 10 und 11,
**dadurch gekennzeichnet,**
**dass** wenigstens zwei Einrichtungen (11) und (12) zur Reinigung des Perkolats vorgesehen sind, wobei hygienisiertes Perkolat aus einem Fermenter (13) in die eine Einrichtung (11) geleitet und nicht hygienisiertes Perkolat aus einem Fermenter (13) in die jeweils andere Einrichtung (12) geleitet wird.

13. Verfahren nach einem der Ansprüche 9 bis 12,
**dadurch gekennzeichnet,**
**dass** im Perkolatspeicher (10) hinter dem Perkolateinlass (10c) auf eine vorgegebene Temperatur eingeregelt wird.

14. Verfahren nach einem der Ansprüche 11 bis 13,
**dadurch gekennzeichnet,**
**dass** wenigstens eine der Einrichtungen (11) und (12) ein Sandfang ist.

15. Verfahren nach einem der Ansprüche 9 bis 14,
**dadurch gekennzeichnet,**
**dass** das Perkolat im Perkolatspeicher (10) und/oder in einer und/oder beiden der Einrichtungen (11, 12) zur Reinigung des Perkolats so beheizt wird, dass eine vorgegebene Mindesttemperatur des Perkolats eingeregelt wird.

## Claims

1. Plant for recovering biogas with at least one fermenter (13) and a percolate tank (10) for collecting the percolate drawn off from the at least one fermenter (13), the percolate tank (10) having a percolate inlet (10c) that can be connected to an outlet of the at least one fermenter (13) and a percolate outlet (10b),
**characterised in**
**that** the flow path (P) in the percolate tank between the percolate inlet (10c) and the percolate outlet (10b) is designed in the form of a meander, a spiral or a screw, so that as a result of the extended flow path (P), a sufficiently long dwell time for the percolate is enabled at a thermophilic adapted temperature, the path length of the flow path of the percolate and the temperature of the percolate on its path between the percolate inlet (10c) and the percolate outlet (10b) being adapted in such a way that only sanitised percolate can be drawn off at the percolate outlet (10b).

2. Plant according to claim 1,
**characterised in**
**that** the percolate tank (10) is arranged beneath the at least one fermenter (13).

3. Plant according to one of the preceding claims,
**characterised in**
**that** a plurality of fermenters (13) is provided, at least one of which is designed for at least intermittent thermophilic operation.

4. Plant according to one of the preceding claims,
**characterised in**
**that** between the at least one fermenter (13) and the percolate tank (10) a device (11) is connected for purifying the percolate, and if necessary, an additional device (12).

5. Plant according to claims 3 and 4,
**characterised in**
**that** at least two devices (11) and (12) are provided for purifying the percolate, fermenters (13) and devices (11) and (12) being connectable in such a way that sanitised percolate coming from one fermenter (13) is guided into the one device (12) and non-sanitised percolate coming from a fermenter (13) is guided into the respective other device (11).

6. Plant according to one of the preceding claims,
**characterised in**
**that** in the percolate tank (10) behind the percolate inlet (10c) at least one heating section (10a) is provided, on which the percolate going into the percolate tank (10) can be heated to a specified temperature.

7. Plant according to one of claims 4 to 6,
**characterised in**
**that** at least one of the devices (11) and (12) is a grit trap.

8. Plant according to one of the preceding claims,
**characterised in**
**that** in the percolate tank (10) and/or one and/or both of the devices (11, 12) for purifying the percolate a heater is provided which can be operated in such a way that a specified minimum temperature of the percolate is regulated.

9. Method for recovering biogas, in which substrate is percolated into at least one fermenter (13) with percolate and the percolate, after percolating, goes into a percolate tank (10), the percolate tank (10) having a percolate inlet (10c) which can be connected with an outlet of the at least one fermenter (13) and a percolate outlet (10b),
**characterised in**
**that** the percolate is guided between the percolate inlet (10c) and the percolate outlet (10b) of the percolate tank (10) along a flow path (P) in the form of a meander, a spiral or a screw, so that as a result of the extended flow path (P) an especially long dwell time for the percolate is enabled at a thermophilic adapted temperature, the path length of the flow path (P) of the percolate and the temperature of the percolate on its path between the percolate inlet (10c) and the percolate outlet (10b) being adjusted in such a way that only sanitised percolate can be drawn off at the percolate outlet (10b).

10. Method according to claim 9,
**characterised in**
**that** a plurality of fermenters (13) is used, at least one of which is operated in thermophilic mode at least part of the time.

11. Method according to one of claims 9 and 10,
**characterised in**
**that** between the at least one fermenter (13) and the percolate tank (10) the percolate is guided through a device (11) for purifying the percolate and if necessary an additional device (12).

12. Method according to claims 10 and 11,
**characterised in**
**that** at least two devices (11) and (12) are provided for purifying the percolate, sanitised percolate being guided out of one fermenter (13) into one device (11) and non-sanitised percolate from a fermenter (13) being guided into the respective other device (12).

13. Method according to one of claims 9 to 12,
**characterised in**
**that** a specified temperature is adjusted in the percolate tank (10) downstream of the percolate inlet (10c).

14. Method according to one of claims 11 to 13,
**characterised in**
**that** at least one of the devices (11) and (12) is a grit trap.

15. Method according to one of claims 9 to 14,
**characterised in**
**that** the percolate in the percolate tank (10) and/or in one and/or both of the devices (11, 12) for purifying the percolate is heated such that a specified minimum temperature of the percolate is regulated.

## Revendications

1. Dispositif pour la production de biogaz, avec au moins un fermenteur (13) et un réservoir de percolat (10) pour la réception du percolat en provenance du fermenteur (13) au moins prévu, sachant que le réservoir de percolat (10) présente une entrée de percolat (10c), qui peut être reliée avec une sortie du fermenteur (13) au moins prévu, et une sortie de percolat (10b),
**caractérisé en ce que**
le parcours d'écoulement (P) s'étend, dans le réservoir de percolat, en forme de méandre, de spirale ou de colimaçon, entre l'entrée de percolat (10c) et la sortie de percolat (10b), de sorte que la durée de séjour du percolat, à une température réglée de manière thermophile soit particulièrement longue, en raison du parcours d'écoulement (P) prolongé, sachant que la longueur du parcours d'écoulement (P) du percolat et la température du percolat, pendant son parcours de l'entrée de percolat (10c) à la sortie de percolat (10b), sont réglées de sorte que le percolat prélevé à la sortie de percolat (10b) est exclusivement un percolat hygiénisé.

2. Dispositif selon la revendication 1,
**caractérisé en ce que**
le réservoir de percolat (10) est disposé au-dessous du fermenteur (13) au moins prévu.

3. Dispositif selon l'une des revendications précédentes,
**caractérisé en ce que**
sont prévus plusieurs fermenteurs (13), dont au moins l'un est conçu pour un fonctionnement thermophile, au moins temporairement.

4. Dispositif selon l'une des revendications précédentes,
**caractérisé en ce**
**qu'**une installation de nettoyage de percolat (11) et, le cas échéant, une installation supplémentaire (12) est / sont connectée(s) entre le fermenteur (13) au moins prévu et le réservoir de percolat (10).

5. Dispositif selon les revendications 3 et 4,
**caractérisé en ce que**
deux installations (11) et (12) sont au moins prévues pour le nettoyage du percolat, sachant que les fermenteurs (13) et les installations (11) et (12) peuvent être connectées de sorte que le percolat hygiénisé, provenant d'un fermenteur (13), est conduit dans une installation (12) et le percolat non hygiénisé, provenant d'un fermenteur (13), est conduit dans l'autre installation (11).

6. Dispositif selon l'une des revendications précédentes,
**caractérisé en ce que**,
dans le réservoir de percolat (10), derrière l'entrée de percolat (10c), est prévu au moins un parcours de chauffage (10a), sur lequel le percolat parvenant au réservoir de percolat (10) peut être chauffé à une température prédéterminée.

7. Dispositif selon l'une des revendications 4 à 6,
**caractérisé en ce que**
l'une des installations (11, 12) au moins prévue est un dessableur.

8. Dispositif selon l'une des revendications précédentes,
**caractérisé en ce que**,
dans le réservoir de percolat (10) et / ou dans une et / ou dans les deux installations (11, 12) de nettoyage du percolat, est prévu un chauffage, qui fonctionne de sorte qu'une température minimum, prédéterminée pour le percolat puisse être réglée.

9. Procédé de production de biogaz, dans lequel un substrat est percolé avec un percolat, dans au moins un fermenteur (13), et ledit percolat parvient, après la percolation, dans un réservoir de percolat (10), sachant que ledit réservoir de percolat (10) présente une entrée de percolat (10c), pouvant être reliée à une sortie du fermenteur (13) au moins prévu, et une sortie de percolat (10b),
**caractérisé en ce que**
le percolat est conduit, entre l'entrée de percolat (10c) et la sortie de percolat (10b) du réservoir de percolat (10), sur un parcours d'écoulement (P) en forme de méandre, de spirale ou de colimaçon, de sorte que la durée de séjour du percolat, à une température réglée de manière thermophile soit particulièrement longue, en raison du parcours d'écoulement (P) prolongé, sachant que la longueur du parcours d'écoulement (P) du percolat et la température du percolat, pendant son parcours de l'entrée de percolat (10c) à la sortie de percolat (10b), soient réglées de sorte que le percolat prélevé à la sortie de percolat (10b) est exclusivement un percolat hygiénisé

10. Procédé selon la revendication 9,
**caractérisé en ce que**
l'on utilise une pluralité de fermenteurs (13) dont l'un au moins est exploité, au moins temporairement, de manière thermophile.

11. Procédé selon l'une des revendications 9 et 10,
**caractérisé en ce que**
le percolat est conduit, entre le fermenteur (13) au moins prévu et le réservoir de percolat (10), à travers une installation de nettoyage (11) de percolat et, le cas échéant, d'une installation supplémentaire (12).

12. Procédé selon les revendications 10 et 11,
**caractérisé en ce que**
deux installations (11) et (12) sont au moins prévues pour le nettoyage du percolat, sachant que le percolat hygiénisé, en provenance d'un fermenteur (13), est conduit dans une installation (12) et le percolat non hygiénisé, en provenance d'un fermenteur (13), est conduit dans l'autre installation (11).

13. Procédé selon l'une des revendications 9 à 12,
**caractérisé en ce que**,
dans le réservoir de percolat (10), derrière l'entrée de percolat (10c), une température prédéterminée peut être réglée.

14. Procédé selon l'une des revendications 11 à 13,
**caractérisé en ce que**
l'une des installations (11, 12) au moins prévue est un dessableur.

15. Procédé selon l'une des revendications 9 à 14,
**caractérisé en ce que**,
dans le réservoir de percolat (10) et / ou dans une et / ou dans les deux installations (11, 12) de nettoyage du percolat, le percolat est chauffé sorte qu'une température minimum prédéterminée du percolat peut être réglée.
